(19) Europäisches Patentamt / European Patent Office / Office européen des brevets

(11) **EP 3 550 318 A1**

(12) **EUROPÄISCHE PATENTANMELDUNG**

(43) Veröffentlichungstag:
**09.10.2019 Patentblatt 2019/41**

(21) Anmeldenummer: **18166126.5**

(22) Anmeldetag: **06.04.2018**

(51) Int Cl.:
**G01R 33/24** (2006.01)  **G01R 33/561** (2006.01)
**G01R 33/56** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder:
• **Körzdörfer, Gregor**
  **91058 Erlangen (DE)**
• **Nittka, Mathias**
  **91083 Baiersdorf (DE)**

Bemerkungen:
Geänderte Patentansprüche gemäss Regel 137(2) EPÜ.

(54) **VERFAHREN ZUR ERZEUGUNG EINER B0-KARTE MITTELS EINES MAGNETRESONANZ-FINGERABDRUCKVERFAHRENS**

(57) Die Erfindung betrifft ein Verfahren zur Erzeugung einer $B_0$-Karte eines Untersuchungsbereiches mit den Schritten: Bereitstellen eines Magnetresonanzdatensatzes mit mehreren Bilddatensätzen, wobei die Bilddatensätze mit wenigstens zwei Messsequenzen aufgenommen wurden und die einander entsprechenden Bildelemente der Bilddatensätze jeweils einen zeitabhängigen Signalverlauf darstellen, und Erzeugen einer $B_0$-Karte des Untersuchungsbereiches aus den Bilddatensätzen, wobei der $B_0$-Wert eines Bildelementes der $B_0$-Karte aus dem zugehörigen Signalverlauf bestimmt wird..

Die Erfindung betrifft ferner ein Computerprogrammprodukt.

Die Erfindung betrifft ferner einen Datenträger.

Die Erfindung betrifft ferner eine Magnetresonanzanlage, mit der das vorgenannte Verfahren ausführbar ist.

FIG 7

EP 3 550 318 A1

**Beschreibung**

Die Erfindung betrifft ein Verfahren zur Erzeugung einer $B_0$-Karte eines Untersuchungsbereiches.

Der Begriff Suszeptibilität beschreibt im Allgemeinen die Magnetisierbarkeit einer Substanz bzw. eines Gewebes. Dabei gibt es bei Patienten in Geweben insbesondere Suszeptibilitätsunterschiede in Abhängigkeit der Gewebezusammensetzung, beispielsweise des Blutanteils. Zu Sprüngen in der Suszeptibilität kommt es an den Grenzflächen zwischen Geweben mit unterschiedlicher Suszeptibilität, z.B. am Übergang von Körpergewebe zur umgebenden Luft oder luftgefüllten Hohlräumen im Körper. Diese Suszeptibilitätssprünge können Signalauslöschungen verursachen.

Dies ergibt sich aus der Verknüpfung der magnetischen Feldstärke B mit der magnetischen Erregung H und der Magnetisierung J:

$$B \; = \; \mu_0 \, (H+J) \, .$$

Da sich die Magnetisierung J auch in Abhängigkeit der Suszeptibilität $\chi$ darstellen lässt als

$$J \; = \; \chi H$$

ergibt sich

$$B \; = \; \mu_0 \mu_r H \; = \; \mu_0 \, (1+\chi) H \; = \; B_0 \; + \; \mu_0 J \, .$$

An der Grenzfläche zweier Gewebe bzw. Stoffe mit unterschiedlichen $\chi$-Werten entsteht daher ein Gradient $\Delta B$:

$$\Delta B \; = \; \mu_0 \, (\chi_1 - \chi_2) \, H$$

Die an den Grenzflächen vorhandenen Zusatzgradienten lassen die Orientierung der signalerzeugenden magnetischen Momente ungleich werden, wodurch es zu Signalverlusten z.B. durch Dephasierung innerhalb des Voxels und Änderungen der Signalphase kommt.

Da sich krankheitsbedingt Blutungen oder auch Kalzifizierungen im Gewebe ergeben können sind Karten über die Suszeptibilitäsverteilung oder Suszeptiblitätssprünge auch von diagnostischem Wert.

Zur Quantifizierung der Suszeptibilität sind unterschiedliche Verfahren bekannt. In Haacke et al., Quantitative susceptibility mapping: current status and future directions, Magnetic Resonance Imaging 33 (2015) S. 1 - 33 sind mehrere Verfahren vorgestellt. Dabei wird immer ein Phasenbild ausgewertet.

Es sind weitere Parameter bekannt, die quantifizierbar sind. Dabei gibt es Parameter, die vom Patienten oder Untersuchungsbereich abhängen, z.B. die Relaxationszeiten $T_1$, $T_2$ und $T_2^*$, die Spindichte, diffusionsabhängige Parameter wie der ADC oder auch Flussgeschwindigkeiten. Aber auch geräteabhängige Größen können eingehen, bspw. die Stärke des Sendefeldes $B_1$.

Einzelnen Geweben sind Durchschnittswerte der Parameter zugeordnet. Weiße Gehirnmasse hat einen bestimmten $T_1$- und $T_2$-Wert. Diese Zuordnung kann zumindest an einem Gerät unter Verwendung immer derselben Messsequenz getroffen werden.

Erkrankungen können eine Veränderung der patientenabhängigen Parameter bewirken. Es existiert daher eine Vielzahl an Studien, die einer Erkrankung Veränderungen eines bestimmten Parameters zuordnen. Verengungen von Gefäßen äußern sich durch eine Erhöhung der Flussgeschwindigkeit, Karzinome verändern die Relaxationszeiten, etc.

Eine Quantifizierung der patientenabhängigen Parameter ist daher ebenfalls wertvoll für die Diagnose. Für die Therapiebegleitung. Es existiert daher eine Vielzahl an Methoden zur Quantifizierung jeweils einzelner Parameter. Alleine für Messung der $T_1$-Relaxationszeit können wenigstens ein Dutzend Verfahren eingesetzt werden. Bei jeder variiert die resultierende $T_1$-Zeit in einem gewissen Rahmen.

Zusammen mit der Geräteabhängigkeit der Gewebeparameter führt das dazu, dass die Aussagekraft der Parameterkarten im Vergleich zur aufzuwendenden Messzeit zu gering ist, um flächendeckend angewendet zu werden. Stattdessen ist es gebräuchlich, gewichtete Bilder aufzunehmen. Das heißt, dass ein Spinecho mit einer kurzen Repetitionszeit $T_R$ eingesetzt wird, um eine $T_1$-Wichtung zu erhalten. Um eine $T_2$-Wichtung zu erzielen wird dagegen eine lange Echozeit $T_E$ verwendet. Auf diese Art und Weise kann man für viele Gewebeparameter Wichtungen vornehmen um in akzeptabler Zeit zu den benötigten Bilddaten zu gelangen. Die gilt auch für die Suszeptibilitätsmessungen. Das Akronym für susceptibility weighted imaging lautet SWI.

Ein neuerer Ansatz, um die Gewebeparameter zu erhalten, ist das sogenannte Magnetresonanzfingerprinting, auch MR Fingerprinting oder MRF abgekürzt. Ma D. et al: Magnetic resonance fingerprinting. Nature 495, S. 187 - 193 (2013) schlagen dabei vor, pseudorandomisierte Messparameter einzusetzen. Dabei werden bei einer bSSFP-Sequenz Messparameter nach der Aufnahme eines Bildes geändert. Variiert werden konkret die Repetitionszeit und der Flipwinkel des Anregungsimpulses. Die Messung erfolgt aber in einem Zug, sodass die AusgangsMagnetisierung nach der Aufnahme des ersten Bildes die Eingangs-Magnetisierung des zweiten Bildes darstellt.

Die so erhaltenen Messsignale ergeben einen Signalverlauf für jedes Bildelement der aufgenommenen Bilder. Dieser Signalverlauf hängt von $T_1$, $T_2$ und $B_0$ ab. $B_0$ ist dabei eine Größe die geräteseitig beinflusst wird, also der Feldverteilung des von außen angelegten statischen Magnetfeldes, sowie lokaler Änderung des Magnetfeldes im Körper durch dessen variable Suszeptibilität.

Um aus den Signalverläufen die Parameter, zwei Gewebeparameter und einen Geräteparameter, zu erhalten wird folgendermaßen vorgegangen:

Zu jedem Parameter wird für einen Satz an vorgegebenen Werten der Signalverlauf simuliert. Es werden also für $T_1$ bspw. in 100 ms-Schritten, für $T_2$ in 10 ms-Schritten und für $B_0$ in 0.1 Hz-Schritten simulierte Signalverläufe erstellt. Nimmt man 50 Werte für $T_1$, 50 für $T_2$ und 100 für $B_0$, so enthält das Dictionary 250.000 Einträge. Nach der Messung wird für jedes Bildelement im Dictionary nachgesehen, welcher der simulierten Signalverläufe am besten auf den gemessenen passt. Dieser Vorgang wird Matching genannt. Die zu dem als am besten passenden Signalverlauf abgespeicherten Werte von $T_1$, $T_2$ und $B_0$ sind dann diejenigen, die für das Bildelement determiniert werden.

Dieses Vorgehen weist mehrere Vorteile auf. Zum einen erhält man mit einer Messung mehrere Parameterkarten. Weiterhin kann neben den Gewebeparametern $T_1$ und $T_2$ auch der Geräteparameter $B_0$ gemessen werden. Dadurch eliminiert man das Problem der Abhängigkeit der Gewebeparameter von diesem Wert.

Ein Nachteil besteht darin, dass bei der bei Ma verwendeten Sequenz durch den langen Spiral-Readout eine untere Grenze für die Repetitionszeit vorliegt, die zu bSSFP-typischen Artefakten bei lokalen $B_0$-Änderungen führt.

Es ist daher Aufgabe der vorliegenden Erfindung, ein Verfahren zur Erzeugung einer $B_0$-Karte anzugeben, das eine hohe SNR-Effizienz aufweist, artefaktfrei ist und damit für die klinische Routine geeignet.

Diese Aufgabe wird gelöst durch ein Verfahren zur Erzeugung einer $B_0$-Karte eines Untersuchungsbereiches mit den Schritten:

- Bereitstellen eines Magnetresonanzdatensatzes mit mehreren Bilddatensätzen, wobei die Bilddatensätze mit wenigstens zwei Messsequenzen aufgenommen wurden und die einander entsprechenden Bildelemente der Bilddatensätze jeweils einen zeitabhängigen Signalverlauf darstellen, und
- Erzeugen einer $B_0$-Karte des Untersuchungsbereiches aus den Bilddatensätzen, wobei der $B_0$-Wert eines Bildelementes der $B_0$-Karte aus dem zugehörigen Signalverlauf bestimmt wird.

Bei bekannten Verfahren zur Erstellung von $B_0$-Karten wird eine einzelne Messsequenz wie Spinecho, Fast Spin Echo, TrueFISP etc. eingesetzt. Bei dieser werden ein oder mehrere Messparameter wie bspw. die Echozeit $T_E$ oder die Repetitionszeit $T_R$ gezielt oder zufällig verteilt variiert. An den oder die variierten Größen werden entweder die Modellfunktionen angefittet oder Übereinstimmungen mit simulierten Signalverläufen gesucht.

Statt einer Messsequenz werden wie beschrieben wenigstens zwei unterschiedliche Messsequenzen eingesetzt. Dadurch können, wie weiter unten ausführlicher beschrieben, Artefakte vermieden und die Güte der erhaltenen $B_0$-Werte gesteigert werden.

Die Auswertung wird wie bei bekannten Methoden bildelementweise vorgenommen. Jeder Bilddatensatz hat eine Vielzahl an Bildelementen. Üblicherweise werden die Bilddatensätze mit einer übereinstimmenden Auflösung, also gleichem FoV und gleicher Bildelementanzahl in allen Richtungen, akquiriert. Dann kann aus einander entsprechenden Bildelementen ein Signalverlauf entweder gegen den variierten Parameter oder gegen die Zeit, aufgetragen werden. Dies geschieht wie gesagt bildelementweise. So kann rein exemplarisch jeweils das Bildelement (23,65) in allen Bilddatensätzen herangezogen werden, um einen Signalverlauf zu erhalten.

Es wird dann für alle Bildelemente oder eine ROI, eine region of interest, die Auswertung vorgenommen, um eine Parameterkarte zu erzeugen. Es ist also nicht zwingend, immer alle Bildelemente auszuwerten.

Wird im Folgenden also von einem Signalverlauf gesprochen wird das prinzipielle Vorgehen angesprochen. Bei Verwendung der Mehrzahl, also Signalverläufen, ist gemeint, dass das Beschriebene jeweils für ein Bildelement, aber insgesamt für mehrere Bildelemente durchgeführt wird.

Die dabei verwendeten Formulierungen beziehen sich auf die rekonstruierten Bilddatensätze und implizieren keine kartesische k-Raum-Abtastung. Dies wird auch bei den Ausführungsformen deutlich, die allesamt auf einer Spiralabtastung des k-Raums beruhen. Eine derartige Abtastung ist aber auch nicht zwingend zur Durchführung des Verfahrens sondern lediglich bevorzugt.

Vorteilhafterweise kann aus der $B_0$-Karte eine Suszeptibilitätskarte abgeleitet werden. Die Suszeptibilitätskarte soll kleinräumige Feldänderungen darstellen, verursacht durch lokale Änderungen des Gewebes, z.B. durch Blutgerinnsel oder auch Kalzifizierung. Nicht dargestellt werden sollen hingegen die großräumigen Feldänderungen, wie sie typischerweise durch Inhomogenitäten des geräteseitigen Grundmagnetfeldes erzeugt werden. Um die großräumigen, langsam variierenden Feldänderungen zu beseitigen, kann auf die $B_0$-Karte ein Lowpass-Filter angewendet werden und von der originalen $B_0$-Karte abgezogen werden, um die Suszeptibilitätskarte zu erhalten. Analog kann auch ein Hochpass-Filter auf die $B_0$-Karte angewendet werden, um die Suszeptibilitätskarte zu erhalten. Somit kann die Suszeptibilitätskarte ohne die Auswertung von Phaseninformationen erhalten werden. Letzten Endes müssen also die hochfrequenten Anteile extrahiert werden.

Es sollte an dieser Stelle beachtet werden, dass die gerade genannten Phaseninformationen das komplexe Messsignal betreffen, bei dem Magnitude und Phasenwert existieren. Weiter unten wird über Phasenzyklen und auch in diesem Zusammenhang von Phasen gesprochen. Dabei ist aber die Auslenkrichtung von Hochfrequenzimpulsen gemeint. Trotz des gleichen Namens sind also ganz unterschiedliche Elemente angesprochen.

Bevorzugt kann aus den Signalverläufen jeweils auch ein $B_1$-Wert ermittelt werden. Mit anderen Worten kann aus dem Magnetresonanzdatensatz auch eine $B_1$-Karte ermittelt werden. Damit weist das vorgeschlagene Verfahren den Vorteil auf, dass dieser Geräteparameter aus dem Experiment als Ergebnis mit hervorgeht und nicht das Experiment so auszugestalten ist, dass er die Messung nicht beeinflusst.

Vorteilhafterweise können bei der Aufnahme des Magnetresonanzdatensatzes wenigstens zwei Abschnitte vorhanden sein und in wenigstens einem Abschnitt Bilddatensätze mit einer TrueFISP-Messsequenz und in wenigstens einem Abschnitt Bilddatensätze mit einer FLASH-Messsequenz aufgenommen werden.

Als Sequenz oder Messsequenz wird wie üblich eine Abfolge von HF-Impulsen, Gradientenfeldern, Wartezeiten und Akquisitionsfenstern bezeichnet, die den Ablauf der Messsequenz genau festlegen und charakterisieren. Beispiele für Messsequenzen sind die bereits angesprochenen FLASH und TrueFisp. Andere Beispiele für Messsequenzen sind Gradientenecho, EPI, Spinecho, TSE (Turbo Spin Echo) usw.

Das eingangs genannte bSSFP ist das Akronym für balanced steady state free-precession und wird auch TrueFISP genannt. Es ist, wie auch FISP, eine Sequenz, die im steady state longitudinale und transversale Magnetisierung verwendet. Im Gegensatz hierzu wird bei FLASH oder SPGR-Sequenzen nur longitudinale Magnetisierung verwendet.

Unter TrueFISP versteht man dabei ein Sequenzdesign, bei dem sich alle Momente nach einer Repetitionszeit $T_R$ zu 0 summieren. Bei FISP ist wenigstens eines der Momente nicht ausbalanciert.

Durch diese Kombination ist es möglich, gleichzeitig und artefaktreduziert die gewünschten Parameter zu erhalten.

Weiterhin können in wenigstens einem Abschnitt Bilddatensätze mit einer FISP-Messsequenz aufgenommen werden. Die Verwendung einer FISP-Messsequenz hilft weiter bei der Reduzierung von Artefakten.

Vorteilhafterweise können die gemessenen Signalverläufe jeweils zur Bestimmung des $B_0$-Wertes oder des $B_0$-Wertes und wenigstens eines weiteren Parameterwertes mit simulierten Signalverläufen verglichen werden. Mit anderen Worten kann also eine Auswertung wie vom MR Fingerprinting her bekannt benutzt werden. Die simulierten Signalverläufe können als Dictionary vorhanden sein. Die beste Übereinstimmung determiniert die gesuchten Parameter.

Mit besonderem Vorteil kann bei der Simulation der Signalverläufe nur ein reduzierter $B_0$-Wertebereich abgedeckt werden. Wie weiter oben beschrieben vergrößert sich die Simulationsbasis mit jedem eingehenden Parameter. Es hat sich herausgestellt dass sich das Dictionary auf eine handhabbare Größe auch unter Einbeziehung von $B_0$ beschränken lässt, indem der eingehende $B_0$-Wertebereich beschränkt wird.

Insbesondere kann der $B_0$-Wertebereich auf Werte von $-(1/T_R)/2$ bis $+(1/T_R)/2$ eingeschränkt werden. Messsignale mit $dB_0 = x + n*(1/T_R)$ unterscheiden sich nur geringfügig von Messsignalen mit $B_0 = x$. Dabei sind x reell und n ganzzahlig. Dementsprechend kann ein Dictionary mit einem beschränkten Umfang aufgebaut werden. Die im Dictionary noch enthaltenen $B_0$-Werte kann man auch als Referenz-$B_0$-Werte bezeichnen.

Natürlich soll die $B_0$-Karte nicht nur Werte aus dem beschränkten Wertebereich der Referenz-$B_0$-Werte aufweisen. Vorzugsweise können die durch den Vergleich ermittelten Referenz-$B_0$-Werte nach Abschluss des Vergleichs mit den simulierten Signalverläufen auf $B_0$-Werte außerhalb des reduzierten Bereichs erweitert werden. Man beschränkt sich also solange auf einen kleineren $B_0$-Wertebereich, wie dies Vorteile bringt und erweitert den Bereich danach wieder. Die beschriebene Beschränkung birgt folgende Vorteile: die Simulation geht schneller, der Speicherplatz für die simulierten Signalverläufe ist geringer und vor allem sinkt die Rechenzeit beim Matching erheblich. Vor allem diese Verringerung ist relevant für die Beschränkung des $B_0$-Wertebereichs.

Vorzugsweise kann eine Erweiterungs-$B_0$-Karte bestimmt werden, indem diese aus zumindest einem Teil der mit der FISP-Messsequenz akquirierten Bilddatensätze berechnet wird. Wie weiter unten beschrieben wird bei der FISP-Messsequenz bevorzugt die Echozeit $T_E$ variiert. Dadurch ist es möglich, aus diesen Bilddatensätzen eine grobe Erweiterungs-$B_0$-Karte zu berechnen. Mit dieser Erweiterungs-$B_0$-Karte können die $B_0$-Werte auf einen $B_0$-Wertebereich transformiert werden, der Werte außerhalb des reduzierten $B_0$-Wertebereichs enthält. Insgesamt kann aber die Zeit für das Matching viel stärker verringert werden als die Nachtragsberechnung verbraucht.

Dies ist möglich, da die Signalverläufe natürlich die vollständigen Informationen enthalten und lediglich aufgrund der vorhandenen Quasi-Periodizität in $B_0$ die Auswertung in dieser Dimension beschränkt werden kann.

Vorteilhafterweise kann aus den Signalverläufen jeweils ein $T_1$-Wert und ein $T_2$-Wert ermittelt werden. Je mehr Werte aus den Signalverläufen ermittelbar sind desto universeller ist das Verfahren einsetzbar. Das beschriebene Vorgehen erlaubt auch die Ermittlung von $T_1$-Werten und $T_2$-Werten.

Bei allen Messsequenzen kann eine kartesische Abtastung des k-Raums eingesetzt werden. Alternativ kann eine radiale Abtastung benutzt werden.

Vorteilhafterweise kann bei wenigstens einer der verwendeten Messsequenzen eine spirale Abtastung des k-Raums verwendet werden. Bevorzugt kann bei allen Messsequenzen und bei der Aufnahme aller Bilddatensätze eine spirale k-Raum-Abtastung verwendet werden.

Dabei kann nach jedem Hochfrequenzimpuls bei einer spiralen Abtastung ein kompletter Bilddatensatz aufgenommen werden. Der Unterschied in den Sequenzen liegt dann nur in den in einer Repetitionszeit $T_R$ angelegten Gradienten.

Bei dieser Ausgestaltung ist die Definition der Repetitionszeit $T_R$ zwar wie gewöhnlich, nämlich die Zeit zwischen zwei korrespondierenden aufeinanderfolgenden Punkten in einer Serie von Hochfrequenzimpulsen und Signalen. Da in einer Repetitionszeit dann auch bei einem TrueFISP nur noch ein Hochfrequenzimpuls auftritt sieht man die Unterschiede zwischen den Sequenzen nicht sofort. Insbesondere liegen Phasenzyklen dann über mehrere Bilddatensätze an und nicht wie bei einer kartesischen Abtastung über mehrere k-Raum-Zeilen.

Nichtsdestotrotz handelt es sich bei den beschriebenen Messsequenzen um diejenigen, die der Name angibt.

Vorteilhafterweise werden bei wenigstens einer Messsequenz die Bilddatensätze ohne steady state aufgenommen. Besonders bevorzugt wird mehr als die Hälfte der Bilddatensätze ohne steady state aufgenommen. Weiterhin können mehr als 75% ohne steady state aufgenommen werden. Vorzugsweise können alle Bilddatensätze ohne steady state aufgenommen werden.

FLASH- und TrueFISP- Bilddatensätze werden bei reiner Bildgebung zumindest zum größten Teil im steady state aufgenommen. Dieser liefert allerdings im Vergleich mehrerer Messungen keine Informationen, da der Signalverlauf sozusagen stagniert.

In der Praxis kann dies bspw. so realisiert sein, dass ein Bilddatensatz mit einer FLASH-Sequenz unter Verwendung eines Flipwinkels von 4° aufgenommen wird und der nächste Bilddatensatz, auch mit einer FLASH-Sequenz akquiriert, mit einem Flipwinkel von 6°, usw.. Bei der später beschriebenen spiralen Abtastung ist dies ausreichend um einen steady state zu verhindern.

Vorzugsweise kann zur Aufnahme der Bilddatensätze ein Empfangsspulenarray verwendet werden. Mit anderen Worten werden die Bilddatensätze mittels paralleler Bildgebung akquiriert. Dann kann der k-Raum undersampelt oder stärker undersampelt als bei einer Spiralabtastung aufgenommen werden.

Eingangs wurde bereits erwähnt, dass eine pseudorandomisierte Verteilung von Messparametern stattfinden kann. Im Unterschied zum bekannten Stand der Technik wird bei dem vorgeschlagenen Verfahren die Repetitionszeit $T_R$ bevorzugt konstant gehalten.

Bei einer kartesischen Abtastung ergeben sich folgende Ausgestaltungen: In einer ersten Ausgestaltung wird sie in einem Bilddatensatz konstant gehalten, kann sich aber bei mehrmaligem Ablaufen der gleichen Messsequenz und erst recht beim Wechsel der Messsequenz ändern. In einer weiteren Ausgestaltung ist die Repetitionszeit $T_R$ auch bei mehrmaligen Durchlaufen der gleichen Messsequenz konstant. Alle FLASH in einem Abschnitt werden also mit der gleichen Repetitionszeit $T_R$ aufgenommen. Die Repetitionszeit $T_R$ kann sich aber bei einem Wechsel zur TrueFISP-Sequenz oder umgekehrt ändern. Sie kann auch in einem späteren Abschnitt bei einer erneuten Verwendung der FLASH-Sequenz differieren. In einer dritten Ausgestaltung ist die Repetitionszeit $T_R$ während der gesamten Messsignalaufnahme konstant.

Bei einer spiralen Abtastung, bei der nach einem Hochfrequenzimpuls ein kompletter Bilddatensatz akquiriert wird, entfällt die erste Ausgestaltung. Dann kann die Repetitionszeit $T_R$ vorzugsweise entweder in jedem Abschnitt oder über alle Abschnitte hinweg konstant gehalten werden.

Dies ist insofern ein Wechsel der Strategie als dass der Signalverlauf nicht durch mehr durch den ständigen Wechsel mehrerer Messparameter sondern u.a. durch einen Wechsel der Messsequenz eine charakteristische Ausprägung erhält.

Dieser Linie folgend können vorteilhafterweise in wenigstens einem Abschnitt Bilddatensätze mit einer FISP-Sequenz aufgenommen werden. Eine FISP-Sequenz beeinflusst den Signalverlauf wieder anders als FLASH oder TrueFISP und trägt so dazu bei, mehr Parameter differenzierbar zu machen.

Vorteilhafterweise kann aus wenigstens einem Signalverlauf wenigstens ein Parameter des Untersuchungsobjektes ermittelt werden. Dies stellt klar, dass das beschriebene Verfahren die Grundlage bildet einen Gewebeparameter, also bspw. $T_1$ oder $T_2$, zu bestimmen, also zu quantifizieren.

Es ist damit bereits implizit angedeutet, dass das beschriebene Verfahren auf der Idee des MR Fingerprinting beruht. Bei herkömmlichen Quantifizierungsverfahren wird eine einzige Messsequenz verwendet und ein einziger Messparameter, bspw. $T_E$, variiert, um so einen Gewebeparameter wie $T_2$ zu bestimmen. Es soll dabei nicht ausgeschlossen werden, mittels des vorgeschlagenen Verfahrens auch auf anderem Weg an Gewebeparameter zu gelangen.

Bevorzugt wird zur Kodierung der zu ermittelnden Parameter bei wenigstens einer Messsequenz als einziger Messparameter der Flipwinkel variiert. Die zu ermittelnden Parameter können wie weiter oben beschrieben $B_0$, $B_1$, $T_1$, $T_2$, ADC, etc. sein. Weder die Repetitionszeit $T_R$ noch die Echozeit $T_E$ werden also geändert. Vorzugsweise wird diese Ausgestaltung bei Akquisitionen mit der FLASH-Messsequenz und/oder TrueFISP-Messsequenz vorgenommen.

Alternativ oder zusätzlich werden bei wenigstens einer Messsequenz als einzige Messparameter der Flipwinkel und die Echozeit $T_E$ variiert.

Bevorzugt kann bei allen Messsequenzen außer der FISP-Messsequenz als einziger Messparameter der Flipwinkel variiert werden. Vorzugsweise können bei der FISP-Messsequenz als einzige Messparameter der Flipwinkel und die Echozeit $T_E$ variiert werden.

Eine kurze Diskussion ist in diesem Zusammenhang bzgl. der Phase der Hochfrequenzimpulse zu führen. Diese ist ebenfalls ein Messparameter und ändert sich bei der Verwendung eines Phasenzyklus wie weiter unten beschrieben. Es ist jedoch eine Größe, die geändert wird, um Geräteunzulänglichkeiten auszugleichen oder die intrinsisch zu einer Messsequenz gehört. Derartige Messparameter werden auch ohne die Quantifizierung von Parametern variiert. Per Definition gehört ein Phasenzyklus demnach nicht zu den zu verändernden Messparametern. Trotzdem sind die beschriebenen Phasenzyklen Bestandteil der Erfindung, und zwar als zweiter veränderlicher Wert neben den Messparametern.

Genauer spezifiziert soll also zur Kodierung der zu ermittelnden Parameter als einziger das Messsignal in Abhängigkeit wenigstens eines Parameters beeinflussender Messparameter der Flipwinkel variiert werden. Anders ausgedrückt soll zur Kodierung der zu ermittelnden Parameter als einziger Messparameter aus der Gruppe Repetitionszeit $T_R$, Echozeit $T_E$ und Flipwinkel $\alpha$ der Flipwinkel $\alpha$ variiert werden. Dies gilt insbesondere für die FLASH- und/oder TrueFISP-Messsequenz.

Alternativ können wie beschrieben bei der FISP-Messsequenz zur Kodierung der zu ermittelnden Parameter als einzige Messparameter aus der Gruppe Repetitionszeit $T_R$, Echozeit $T_E$ und Flipwinkel $\alpha$ der Flipwinkel $\alpha$ und die Echozeit $T_E$ variiert werden.

Der Flipwinkel kann über mehrere Bilddatensätze hinweg einer vorgegebenen Verteilung folgen. Er ist damit nicht mehr pseudorandomisiert sondern mit einer Strategie variiert. In einer Ausgestaltung kann der Flipwinkel einer Normalverteilung folgen. Er beginnt also mit kleinen Flipwinkeln. Der Flipwinkel steigt bis zu einem Maximalwert an und fällt dann wieder ab.

In einer anderen Ausgestaltung ist die Verteilung wie eine halbe Sinuskurve, insbesondere die positive Hälfte, ausgestaltet. Sie steigt im Vergleich zu einer Normalverteilung steiler an und hat im Maximalbereich ein breiteres Plateau.

In einer weiteren Ausgestaltung ist die Verteilung wie eine $\sin^2$-Kurve ausgestaltet. Sie steigt im Vergleich zu einer Sinuskurve noch steiler an.

Vorzugsweise findet sich in einem Abschnitt wenigstens eine Verteilung. Insbesondere findet sich in wenigstens einem Abschnitt genau eine Verteilung. Weiterhin können sich in einem Abschnitt genau zwei Verteilungen finden.

Vorteilhafterweise kann in einem Abschnitt, in dem Bilddatensätze mit einer FLASH-Messsequenz akquiriert werden, einer Normalverteilung gefolgt werden.

Bevorzugt können die Flipwinkel in einem Abschnitt, in dem Bilddatensätze mit einer FISP- oder TrueFISP-Messsequenz aufgenommen werden, einer $\sin^2$-Verteilung folgen.

Vorzugsweise wird ein Teil der Bilddatensätze pausenfrei hintereinander gemessen. Mit anderen Worten kann die Ausgangsmagnetisierung von der folgenden Sequenz übernommen werden. Es geht wie bereits beschrieben gerade darum, Signalverläufe zu generieren, die in Abhängigkeit der Messsequenzen und der zu ermittelnden Parameter Variationen aufweisen. Diese fallen bei Messpausen geringer aus, da das akquirierte Messsignal dann stärker von der Ausgangsmagnetisierung $M_0$ abhängt als von den vorher durchlaufenen Abschnitten.

Vorteilhafterweise werden alle Bilddatensätze in einem Abschnitt pausenfrei hintereinander gemessen. Zwischen zwei Abschnitten ist dann eine grundsätzlich beliebig lange Pause.

Bevorzugt werden alle Messsequenzen bzw. Bilddatensätze, also auch bei einem Messsequenzwechsel bzw. Abschnittswechsel, pausenfrei hintereinander gemessen.

Beispielsweise kann man ein FLASH mit einem großen Flipwinkel bei verhältnismäßig kleinem $T_R$ zur Aufnahme eines Bilddatensatzes verwenden. Bereits dies erlaubt zwar Aussagen über $T_1$. Diese werden aber besser, wenn die nachfolgende Messung, ein FLASH mit einem kleineren Flipwinkel, mit der Magnetisierung am Ende des vorhergehenden FLASH startet als wenn durch eine Pause die Magnetisierung wieder auf $M_0$ relaxiert ist.

Auch stellt eine Messung mit Pausen keinen Signalverlauf mehr dar sondern nur einzelne Messpunkte.

Aus der Verwendung mehrerer Messsequenzen allein ergibt sich allerdings noch nicht der optimale Ablauf, um mit möglichst wenig Bilddatensätzen im Magnetresonanzdatensatz auszukommen. Es hat sich als bevorzugte Ausgestaltung ergeben in drei aufeinanderfolgenden Abschnitten erst eine FISP-Sequenz, dann eine TrueFISP-Sequenz und dann eine FLASH-Sequenz zu verwenden. Das heißt, dass zuerst mehrere FISP-Bilddatensätze aufgenommen werden, wobei in einem Abschnitt grundsätzlich beliebig viele Bilddatensätze aufgenommen werden können, solange es mit der gleichen Messsequenz erfolgt. Dies bedeutet aber nicht, dass in einem Abschnitt alle Messparameter der Sequenz gleich bleiben müssten. Hier darf es eine Variation bspw. zur Verhinderung eines steady state geben. Bevorzugt ist, wie bereits ausführlich beschrieben, den Flipwinkel zu variieren.

Dann folgt ein Abschnitt, in dem mehrere Bilddatensätze mit einer TrueFISP-Sequenz aufgenommen werden und dann ein Abschnitt mit einer FLASH-Sequenz. Auch im letzten Abschnitt werden mehrere Bilddatensätze mit der FLASH-Sequenz akquiriert.

Diese Abfolge aus FISP-, TrueFISP- und FLASH-Sequenz wird im Folgenden als Block bezeichnet.

Vorteilhafterweise kann die beschriebene Abfolge, also der Block, wenigstens einmal wiederholt werden. Der Block wird also wenigstens zweimal durchlaufen. Bevorzugt wird die Abfolge genau dreimal durchlaufen.

Vorteilhafterweise werden mit jeder Sequenz wenigstens 10 Bilddatensätze aufgenommen. Vorzugsweise werden pro Abschnitt wenigstens 10 Bilddatensätze aufgenommen. Bei drei Blöcken hat man dann wenigstens 90 Bilddatensätze. Dies ist eine erheblich größere Anzahl an Stützstellen im Signalverlauf als bei herkömmlichen Parameterkarten, bei denen man aus Messzeitgründen oft lediglich sechs bis zehn Stützstellen hat.

Vorteilhafterweise kann die TrueFISP-Sequenz in wenigstens einem Abschnitt wenigstens einen Phasenzyklus aufweisen. Unter einem Phasenzyklus versteht man die vorgegebene Abfolge der Phase bestimmter oder aller Hochfrequenzimpulse. Dies ist eine vom Flipwinkel unabhängige Größe.

Bevorzugt handelt es sich um einen 180°-Phasenzyklus. Dann wechseln sich die Phasen der Hochfrequenzpulse der TrueFISP-Sequenz von x nach -x oder von y nach -y und umgekehrt ab.

Alternativ kann der Phasenzyklus als 90°-Phasenzyklus ausgestaltet sein. Dann können die Phasen zum Beispiel von x nach y nach -x nach -y und dann wieder von vorn wechseln.

Weiter alternativ kann der Phasenzyklus als 270°-Phasenzyklus ausgestaltet sein. Dabei wird vorzugsweise pro Verteilung ein Phasenzyklus verwendet. Bei drei Verteilungen des Flipwinkels können also auch drei unterschiedliche Phasenzyklen zu Einsatz kommen. Bei mehr Verteilungen können auch mehr Phasenzyklen verwendet werden.

Vorteilhafterweise können in einem Abschnitt bei der TrueFISP-Sequenz zwei Phasenzyklen verwendet werden. Als ein Phasenzyklus kann ein 180°-Phasenzyklus und als ein anderer Phasenzyklus ein 90°-Phasenzyklus verwendet werden. Vorzugsweise kann der 180°-Phasenzyklus als erster Phasenzyklus eingesetzt werden. Durch den Wechsel des Phasenzyklus können zyklusbedingte Artefakte wie die Position des "dark band" variiert oder vermieden werden.

Vorteilhafterweise wird bei der FISP-Sequenz eine vorgegebene Phase verwendet. Bevorzugt wird jedesmal wenn eine FISP-Sequenz aufgenommen wird die gleiche Phase verwendet.

Vorteilhafterweise kann bei Verwendung der FLASH-Sequenz ein RF-Spoiling verwendet werden. RF-Spoiling bedeutet, dass ein Phasenzyklus verwendet wird, mit dem eine mögliche $T_2$-Wichtung der Magnetisierung vermieden wird. Bevorzugt kann die Zusatz-Phase 117° oder ein Vielfaches davon betragen. Die zu verwendende Phase ergibt sich dabei aus der vorhergehenden Phase durch Addition eines Vielfachen von 117°. Die allererste Phase kann beliebig gewählt werden und muss kein Vielfaches von 117° sein. Zu den Vielfachen zählt auch 117° als einfaches Vielfaches.

Die Lösung der eingangs genannten Aufgabe wird außerdem durch ein Computerprogrammprodukt bzw. ein Computerprogramm erzielt, das zur Steuerung einer Steuerungseinrichtung eingesetzt werden kann, welche eine Bilderzeugungseinheit einer Magnetresonanzanlage steuert, welches das vorgenannte erfindungsgemäße Verfahren ausführt.

Daneben betrifft die Erfindung einen Datenträger für eine Steuerungseinrichtung zur Steuerung einer Datenerzeugungseinheit einer Magnetresonanzanlage mit Daten zum Durchführen des beschriebenen Verfahrens. Vorteilhafterweise kann die Datenerzeugungseinheit eine Bilderzeugungseinheit sein.

Daneben betrifft die Erfindung eine Magnetresonanzanlage mit einer Steuerungseinrichtung. Die Magnetresonanzanlage zeichnet sich dadurch aus, dass die Steuerungseinrichtung zur Durchführung des Verfahrens wie beschrieben ausgebildet ist.

Die Implementierung der vorgenannten Verfahren in der Steuervorrichtung kann dabei als Software oder aber auch als (fest verdrahtete) Hardware erfolgen.

Weitere vorteilhafte Ausgestaltungen der erfindungsgemäßen Magnetresonanzanlage korrespondieren zu entsprechenden Ausgestaltungen des erfindungsgemäßen Verfahrens. Zur Vermeidung unnötiger Wiederholungen wird somit auf die entsprechenden Verfahrensmerkmale und deren Vorteile verwiesen.

Weitere Vorteile, Merkmale und Besonderheiten der vorliegenden Erfindung ergeben sich aus der nachfolgenden Beschreibung vorteilhafter Ausgestaltungen der Erfindung.

Dabei zeigen:

Fig. 1      eine Magnetresonanzanlage,

Fig. 2      eine FLASH-Messsequenz,

Fig. 3      eine FISP-Messsequenz,

Fig. 4      eine TrueFISP-Messsequenz, und

Fig. 5      ein Aufnahmeverfahren mit mehreren Messsequenzen,

Fig. 6-8    einen Magnetresonanzdatensatz, und

Fig. 9      eine $B_0$- und eine Suszeptibilitätskarte.

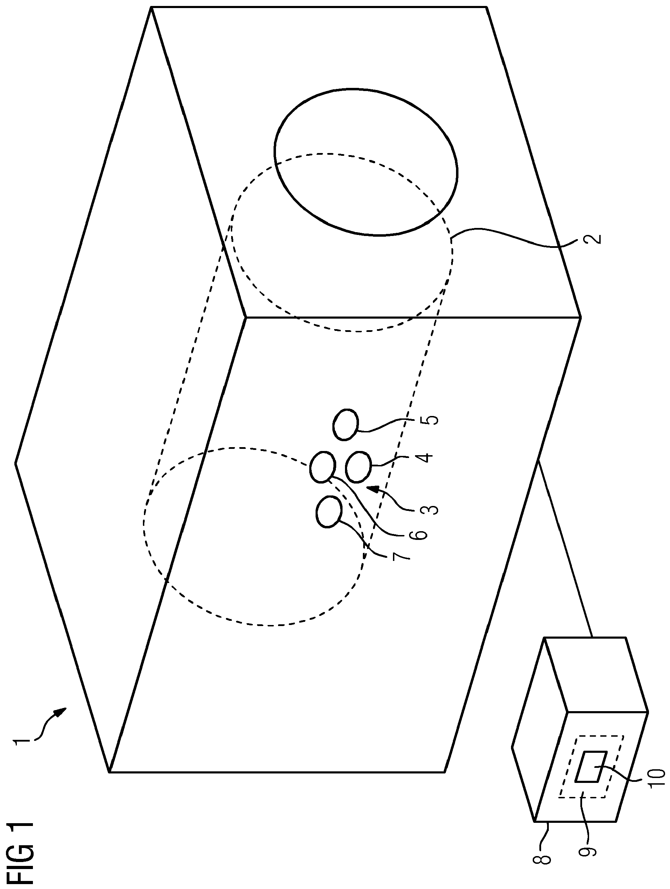

Figur 1 zeigt eine Magnetresonanzanlage 1 mit einer Sendespulenanordnung 2. Die Sendespulenanordnung 2 kann als Bodycoil ausgestaltet sein. Es kann sich dabei aber auch um ein Sendespulenarray handeln. Die Sendespulenanordnung 2 ist gestrichelt dargestellt.

Zur Datenakquisition besitzt die Magnetresonanzanlage 1 eine Empfangsspulenanordnung 3. Die Empfangsspulenanordnung 3 ist vorzugsweise ein Spulenarray mit Spulen 4, 5, 6 und 7. Die Spulen 4, 5, 6 und 7 lesen Messsignale gleichzeitig und damit parallel aus.

Zur Steuerung der Experimente weist die Magnetresonanzanlage 1 eine Steuerungseinrichtung 8 auf.

Die Magnetresonanzanlage 1 besitzt weiterhin einen Datenträger 9 als Teil der Steuerungseinrichtung 8 oder unabhängig davon, auf dem Computerprogramme 10 zur Durchführung von Magnetresonanzmessungen abgespeichert sind.

Weitere Bestandteile der Magnetresonanzanlage 1 wie bspw. Gradientenspulen oder eine Patientenliege sind der Übersichtlichkeit halber nicht dargestellt.

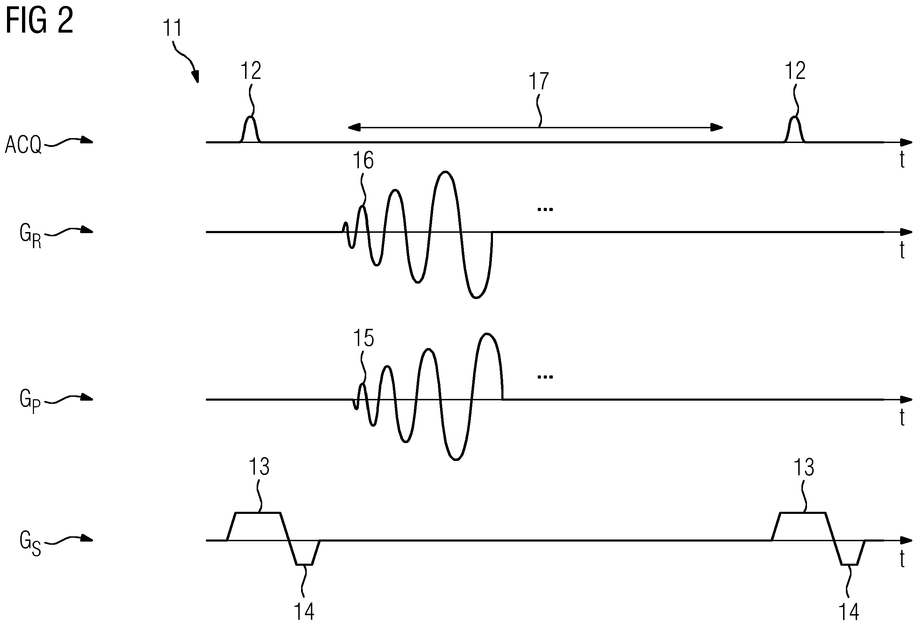

Figur 2 zeigt eine FLASH-Messsequenzdiagramm 11. Die Gradientenachsen sind wie üblich mit $G_R$ für die Leserichtung, $G_P$ für die Phasenkodierrichtung und $G_S$ für die Schichtauswahlrichtung beschriftet. ACQ bezeichnet die Achse für die Hochfrequenzimpulse und die Akquisitionsfenster.

Ein FLASH ist eine gradientenechobasierte Sequenz mit einem Hochfrequenzimpuls 12, dessen Flipwinkel kleiner als 90° ist. Über die Echozeit $T_E$ kann ein $T_2$*-Kontrast eingestellt werden und über die Repetitionszeit $T_R$ ein $T_1$-Kontrast. Der Hochfrequenzimpuls hat typischerweise für gewichtete Messungen einen Flipwinkel zwischen 4° und 30°.

Um mit dem Hochfrequenzimpuls 12 nur eine Schicht anzuregen liegt gleichzeitig zum Hochfrequenzimpuls 12 in Schichtauswahlrichtung $G_S$ ein Schichtauswahlgradient 13 an. Um dessen dephasierende Wirkung auf die Magnetisierung in der Transversalebene zu kompensieren folgt direkt auf den Schichtauswahlgradienten 13 ein Schicht-Rephasiergradient 14.

In Phasenkodierrichtung $G_P$ wird ein Phasenkodiergradient 15 verwendet. Dieser ist, wie der Lesegradient 16 in Leserichtung $G_R$, oszillierend angelegt. Dies wird bevorzugt vorgenommen, um den k-Raum spiralförmig abzutasten. Stattdessen könnte, wie weiter oben bereits beschrieben, auch eine kartesische oder eine radiale Abtastung vorgenommen werden.

Entsprechend können Messsignale 17 akquiriert werden.

Sehr wichtig ist in diesem Zusammenhang, dass in der Repetitionszeit $T_R$ ein gesamter Bilddatensatz aufgenommen wird. Bei der FLASH-Messsequenz 11 - wie auch den weiteren besprochenen Messsequenzen - handelt sich also um eine Art singleshot-Sequenz, da ein einziger Hochfrequenzimpuls 12 ausreicht, um einen kompletten Bilddatensatz zu erhalten.

Der derart akquirierte Rohdatensatz kann durch eine nichtuniforme Fouriertransformation in einen Bilddatensatz umgewandelt werden. Dieser kann artefaktbehaftet sein, für das Matching ist dies ausreichend.

Der zweite Hochfrequenzimpuls 12 rechts im Bild zeigt, dass nach der Aufnahme des ersten Bilddatensatzes ohne Pause der zweite Bilddatensatz begonnen wird. Der zweite Hochfrequenzimpuls 12 kann, wie später ausführlicher beschrieben wird, einen anderen Flipwinkel aufweisen als der erste Hochfrequenzimpuls 12. Weiterhin kann die Phase zur Realisierung eines Phasenzyklus wechseln.
SNR-Probleme können durch parallele Bildgebung verringert werden, da dabei weniger k-Raum-Daten aufzunehmen sind und dadurch die Repetitionszeit $T_R$ verkürzt werden kann.

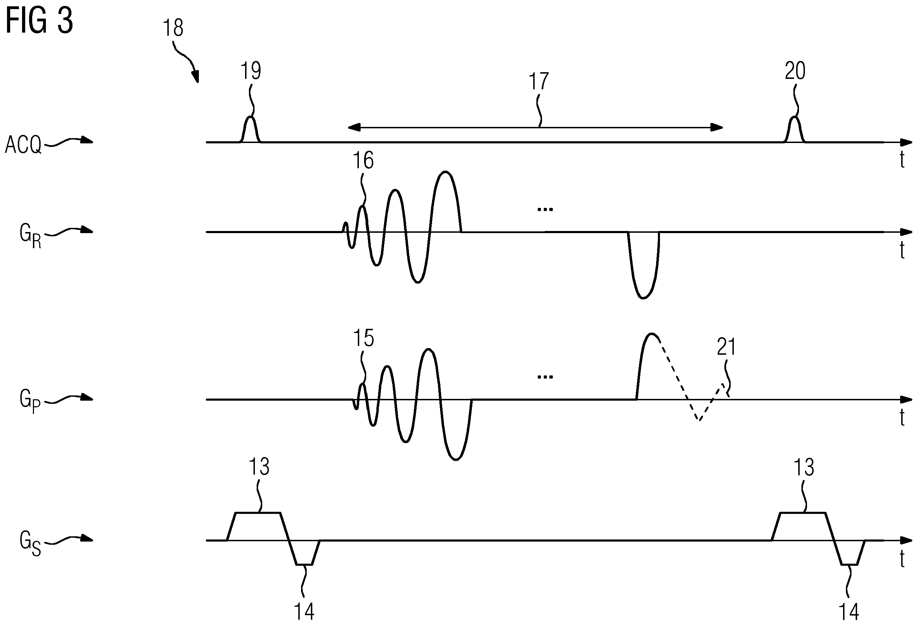

Figur 3 zeigt eine FISP-Messsequenz 18. Bei dieser wird zur Aufnahme eines kompletten Bilddatensatzes ebenfalls nur ein Hochfrequenzimpuls 19 eingesetzt.

Ein Schichtauswahlgradient 13, ein Schicht-Rephasiergradient 14, Phasenkodiergradient 15 und ein Lesegradient 16 liegen wie zur FLASH-Messsequenz 11 angegeben an.

Zusätzlich ist ein Phase-Rewindgradient 21 vorhanden. Dieser sorgt dafür, dass über eine Repetitionszeit $T_R$ die Summe der Gradientenmomente in Phasenrichtung gleich Null ist.

In Schichtrichtung $G_S$ ist über eine Repetitionszeit $T_R$ die Summe der Gradientenmomente nicht Null.

In Leserichtung $G_R$ sind die Gradienten balanciert, dies ist aber nicht zwingend. Die Summe der Gradienten in Leserichtung $G_R$ kann über eine Repetitionszeit hinweg also auch ungleich 0 sein. Da Spiraltrajektorien aufgenommen werden ist das sich ergebende Summenmoment immer gleich, da die einzelnen Gradientenmomente über eine Repetitionszeit hinweg immer den gleichen Verlauf haben.

Der zweite Bilddatensatz wird mit dem Hochfrequenzimpuls 20 begonnen. Dieser besitzt vorzugsweise die gleiche Phase aber einen anderen Flipwinkel als der vorhergehende Hochfrequenzimpuls 19.

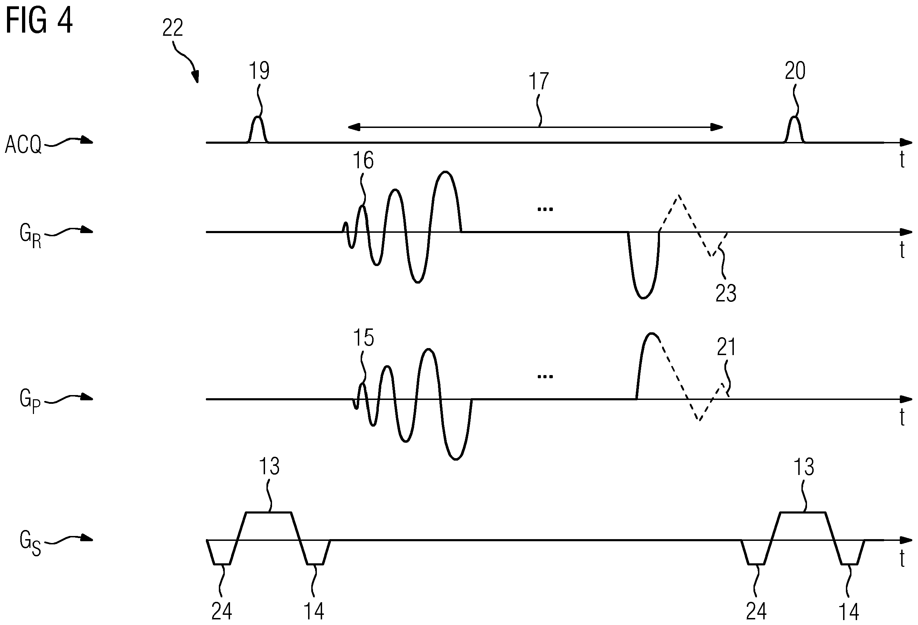

Figur 4 zeigt eine TrueFISP-Messsequenz 22. Grundsätzlich kann dabei auf die Ausführungen zur FLASH-Messsequenz 11 und auch zur FISP-Messsequenz 18 verwiesen werden.

In Ergänzung zu den bereits erwähnten Bestandteilen finden sich bei der TrueFISP-Messsequenz 22 ein Lese-Rewindgradient 23 und ein Schicht-Dephasiergradient 24. Dadurch ist die TrueFISP-Messsequenz 22 über eine Repetitionszeit $T_R$ "fully balanced", d.h. es sind über eine Repetitionszeit $T_R$ die Summen der Gradientenmomente in allen Richtungen gleich Null.

Auch bei der TrueFISP-Messsequenz 22 variieren die Beträge der Flipwinkel der Hochfrequenzimpulse 19 und 20.

Die TrueFISP-Messsequenz 22 kann wie bereits beschrieben Phasenzyklen aufweisen. Wie bereits beschrieben kann ein 90°-Phasenzyklus benutzt werden. Dann hat der erste Hochfrequenzimpuls 19 eine Phase φ, der zweite Hochfrequenzimpuls 20 eine Phase φ+180°, der dritte Hochfrequenzimpuls 25 eine Phase (φ+ 90°), der vierte Hochfrequenzimpuls eine Phase (φ+ 270°), der fünfte Hochfrequenzimpuls eine Phase (φ+ 180°), der sechste Hochfrequenzimpuls eine Phase (φ+ 360°), usw.

Ein 180°-Phasenzyklus springt dann mit 180°-Schritten statt mit 90°-Schritten und ein 270°-Phasenzyklus mit 270°-Schritten.

Bei allen Messsequenzen 11, 18 und 22 ist das Schema zur Aufnahme eines Bilddatensatzes und der Hochfrequenzimpuls samt Gradienten in Schichtauswahlrichtung $G_S$ des nächsten Bilddatensatzes gezeigt, um den Ablauf zu verdeutlichen.

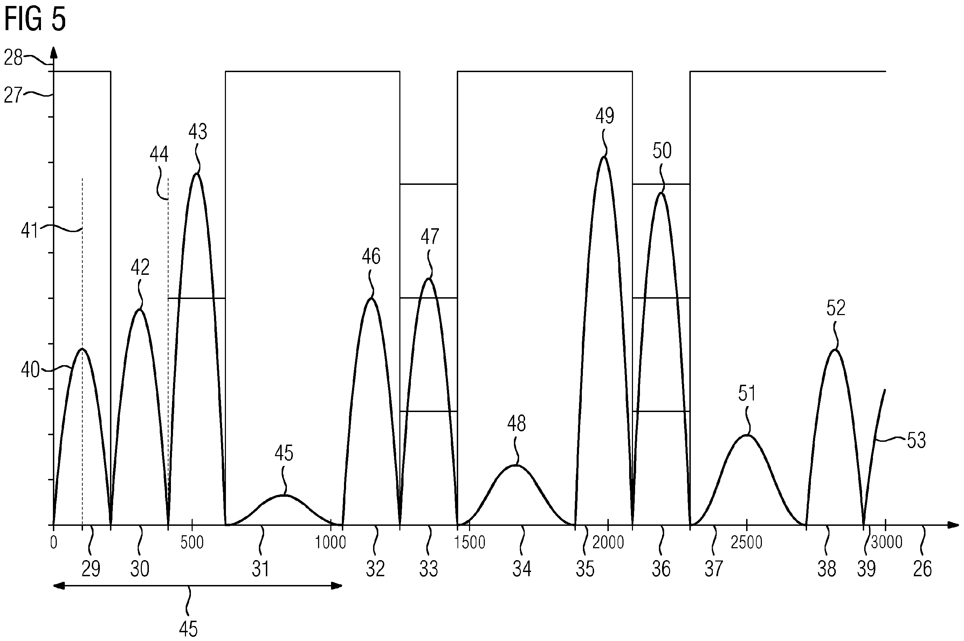

Figur 5 zeigt schematisch ein Aufnahmeverfahren zur Aufnahme eines Magnetresonanzdatensatzes. Dabei sind auf der Achse 26 die Nummer des aufgenommenen Bilddatensatzes und auf der Achse 27 unterschiedliche Größen aufgetragen. Als erste Größe ist der Flipwinkel in ° von 0° beim Ursprung bis 90° beim Achsenpunkt 28 aufgetragen. Die Achse 26 läuft vom Bilddatensatz 1 bis zum Bilddatensatz 3100.

Die 3000 Bilddatensätze sind auf elf Abschnitte 29, 30, 31, 32, 33, 34, 35, 36, 37, 38 und 39 verteilt.

Im ersten Abschnitt 29 ist über die Kurve 40 für zweihundert Bilddatensätze der Flipwinkel der FISP-Messsequenz 18 aufgetragen, der bei der Aufnahme verwendet wurde. Wie zu Figur 3 beschrieben wird nach dem Anlegen eines Hochfrequenzimpulses mit einem bestimmten Flipwinkel ein kompletter Bilddatensatz aufgenommen und dann der nächste Hochfrequenzimpuls mit dem nächsten Flipwinkel angelegt und ein weiterer Bilddatensatz aufgenommen. Figur 5 zeigt in Abschnitt 29 dementsprechend eine Flipwinkelverteilung, die einer $\sin^2$-Kurve entspricht. Der maximale Flipwinkel ist 24° groß und es werden konstante Phasen verwendet.

Für den hundertsten Bilddatensatz ist rein exemplarisch eine Linie 41 eingetragen. Der korrespondierende Flipwinkel ist der maximale Flipwinkel der Kurve 40.

Im zweiten Abschnitt 30 werden vierhundert Bilddatensätze mit der TrueFISP-Sequenz 22 gemäß Figur 4 akquiriert. Dabei werden Flipwinkel gemäß der Kurven 42 und 43 eingesetzt. Bei der Kurve 42 reichen diese bis 45° und bei der Kurve 43 bis 72°.

Auch für den Abschnitt 30 ist rein exemplarisch beim Flipwinkel für den vierhundertsten Bilddatensatz eine Linie 44 eingezeichnet. Hier beträgt der Flipwinkel 1°.

Eine Besonderheit stellt im Abschnitt 30 die Verwendung zweier unterschiedlicher Phasenzyklen dar. Beim Durchlaufen der Flipwinkel der Kurve 42 wird ein 00-Phasenzyklus bzw. kein Phasenzyklus verwendet und beim Durchlaufen der Kurve 43 ein 180°-Phasenzyklus. Ein 00-Phasenzyklus bezeichnet eine feststehende Phase.

Im folgenden Abschnitt 31 sind in der Kurve 45 die Flipwinkel zur Aufnahme von vierhundertfünfzig Bilddatensätzen mit einer FLASH-Sequenz 11 angegeben. Diese sind kleiner als in der FISP- oder TrueFISP-Sequenz und laufen bis 6°. Auch ihre Verteilung ist eine $\sin^2$-Verteilung.

Zusätzlich zur Variation der Flipwinkel wird beim wiederholten Durchlaufen der FLASH-Sequenz ein Phasenzyklus zur Realisierung eines RF-Spoiling angelegt. Dabei wird wie beschrieben die Phase um Vielfache von 117° erhöht.

Die Abfolge der Messsequenzen 11, 18 und 22 bilden zusammen einen Block 45. Dieser wird in Figur 5 insgesamt dreimal verwendet. Dabei wird allein auf den Typ der Messsequenz aber nicht auf die Anzahl der Bilddatensätze oder die Flipwinkelkurven abgestellt.

Im Abschnitt 32 werden wieder 200 Bilddatensätze mit einer FISP-Sequenz 18 aufgenommen. Die Phase ist wie in Abschnitt 29 konstant, aber der maximale Flipwinkel beträgt 45°. Diese liegen auf der Kurve 46.

In Abschnitt 33 folgen 200 Bilddatensätze, die mit einer TrueFISP-Sequenz 22 zu akquirieren sind. Hier wird ein 90°-Phasenzyklus eingesetzt, der maximale Flipwinkel liegt bei 50°. Die Flipwinkel sind auf der Kurve 47 aufgetragen.

Die nächsten ca. 450 Bilddatensätze in Abschnitt 34 sind, wie in Abschnitt 31, mit einer FLASH-Sequenz aufzunehmen. Die Kurve 48 zeigt eine $\sin^2$-Verteilung mit einem Maximalwert von 14°.

Kurve 49 in Abschnitt 35 läuft bis 72° und zeigt die Flipwinkel des Hochfrequenzimpulses 19 beim drittmaligen Verwenden einer FISP-Sequenz 18. Auch in diesem Durchlauf ist die Phase konstant.

Beim Akquirieren weiterer zweihundert Bilddatensätze mit einer TrueFISP-Sequenz 22 gemäß Figur 4 wird ein 270°-Phasenzyklus eingesetzt. Die Flipwinkel, die in der Kurve 50 aufgetragen sind, laufen bis 65°.

Die nächsten ca. 450 Bilddatensätze in Abschnitt 37 werden mit der FLASH-Sequenz 11 gemäß Figur 2 aufgenommen. Die Kurve 51 stellt einen Flipwinkelverlauf bis maximal 20° dar, wiederum $sin^2$-verteilt.

Im letzten Abschnitt 38 liegen zwei Kurven 52 und 53 zur Aufnahme von Bilddatensätzen mit einer FISP-Sequenz. Diese stellen wiederum Flipwinkelverläufe dar. Wie bereits in den vorhergehenden Abschnitten wird bei der FISP-Messsequenz 18 eine konstante Phase verwendet.

Zusammenfassend kann man festhalten, dass, unabhängig von der konkreten Bildanzahl und der jeweils maximalen Flipwinkeln, in allen Abschnitten vorzugsweise ein $sin^2$-verteilter Flipwinkelverlauf verwendet wird. Wie weiter oben beschrieben können in einem Abschnitt auch erheblich weniger Bilddatensätze aufgenommen werden, bevorzugt aber wenigstens 10.

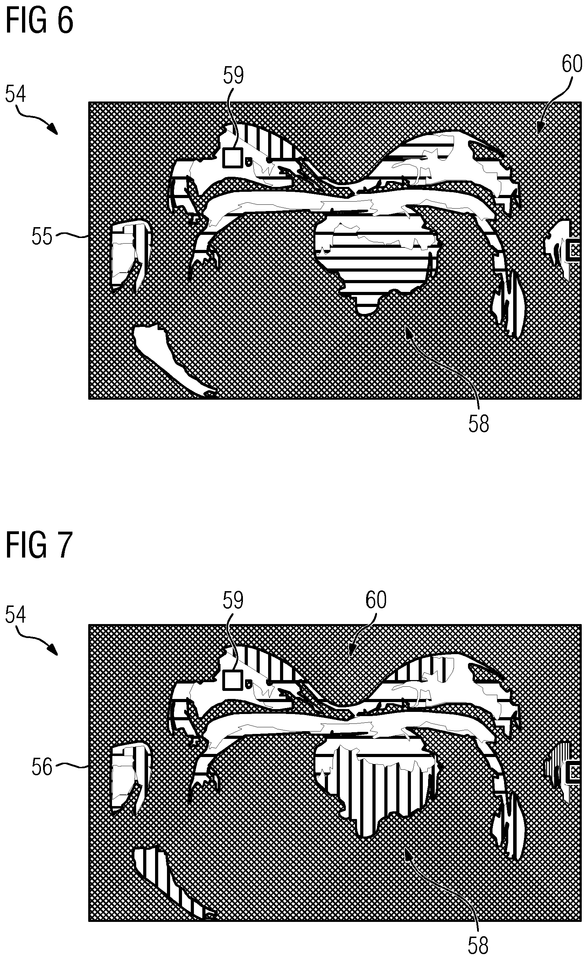

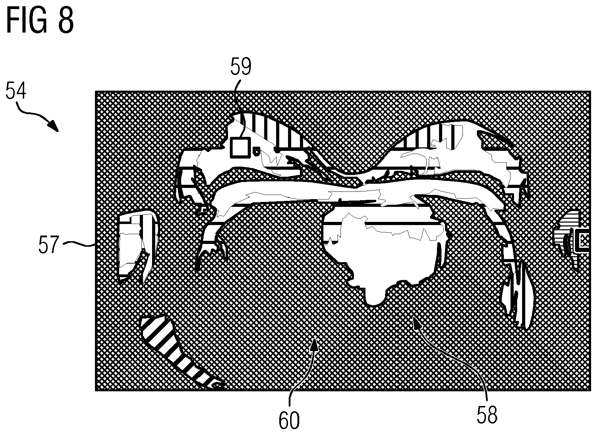

Die Figuren 6 bis 8 zeigen einen Magnetresonanzdatensatz 54 mit Bilddatensätzen 55, 56 und 57. Diese sind exemplarisch für die 3000 Bilddatensätze, die bei dem Verfahren nach Figur 5, erhalten werden. Das notwendige Postprocessing ist soweit bekannt.

Die Bilddatensätze 55, 56 und 57 bilden jeweils einen Untersuchungsbereich 58 ab. Der Bilddatensatz 55 ist mit der FISP-Messsequenz 18 aufgenommen worden, Bilddatensatz 56 mit dem TrueFISP-Messsequenz 22 und Bilddatensatz 57 mit der FLASH-Sequenz 11. Der Flipwinkel ist jeweils einer der möglichen Flipwinkel aus den Kurven 40 bis 53. Das Signal hängt aber auch jeweils von der durchlaufenen Historie ab.

Bei der Auswertung wird bildelementweise vorgegangen. Rein exemplarisch ist das Bildelement 59 eingezeichnet. In allen Bilddatensätzen 55 bis 57 wird das Bildelement an der gleichen Stelle, nämlich das Bildelement 59, herangezogen, um einen Signalverlauf zu erhalten. Für die anderen Bildelemente wird jeweils ein Signalverlauf ermittelt und ausgewertet. Die Bereiche 60, in denen nur Rauschsignal vorhanden ist, können zur Minimierung der Auswertezeit bspw. anhand eines Schwellwertes erkannt und ausgelassen werden.

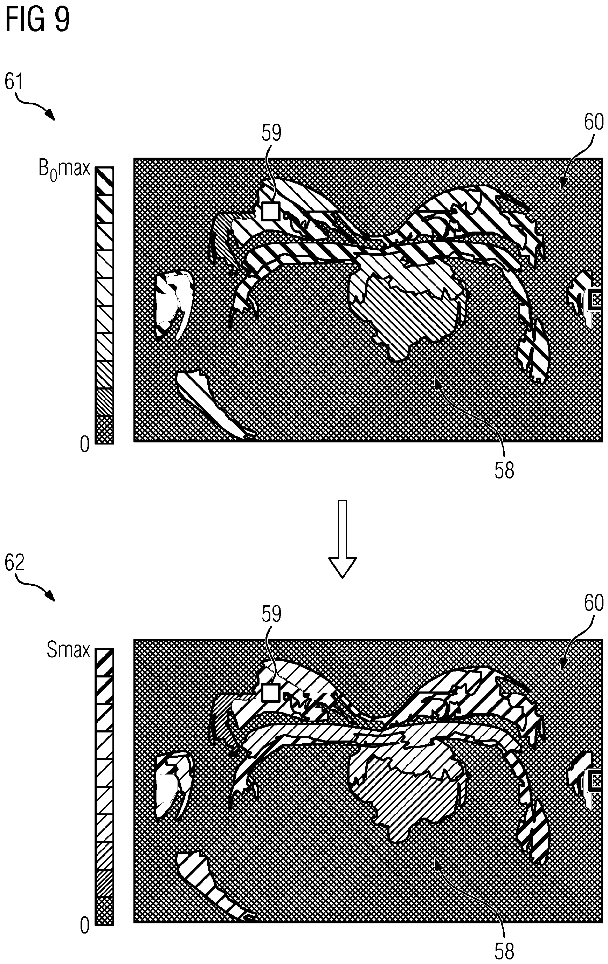

Figur 9 zeigt eine $B_0$-Karte 61, die aus dem Magnetresonanzdatensatz 54 ermittelbar ist. Durch die bildelementweise Auswertung kann für jedes Bildelement 59 wie weiter oben beschrieben ein $B_0$-Wert, ein $B_1$-Wert, ein $T_1$-Wert und ein $T_2$-Wert ermittelt werden. Auch andere Parameter sind grundsätzlich möglich. Aus allen ermittelten $B_0$-Werten kann dann die $B_0$-Karte 61 zusammengesetzt werden, wie es bei der Erstellung von Parameterkarten üblich ist.

Dabei kann die $B_0$-Karte 61 wie beschrieben über eine Referenz-$B_0$-Karte und eine Erweiterungs-$B_0$-Karte berechnet werden.

Aus der $B_0$-Karte 61 kann mittels eines Lowpass-Filters eine Suszeptibilitätskarte 62 berechnet werden. Dadurch kann die Verwendung von Phasenkarten vermieden werden.

## Patentansprüche

1. Verfahren zur Erzeugung einer $B_0$-Karte (61) eines Untersuchungsbereiches (58) mit den Schritten:

   - Bereitstellen eines Magnetresonanzdatensatzes (54) mit mehreren Bilddatensätzen (55, 56, 57), wobei die Bilddatensätze (55, 56, 57) mit wenigstens zwei Messsequenzen (11, 18, 22) aufgenommen wurden und die einander entsprechenden Bildelemente (59) der Bilddatensätze (55, 56, 57) jeweils einen zeitabhängigen Signalverlauf darstellen, und
   - Erzeugen einer $B_0$-Karte (61) des Untersuchungsbereiches (59) aus den Bilddatensätzen (55, 56, 57), wobei der $B_0$-Wert eines Bildelementes (59) der $B_0$-Karte (61) aus dem zugehörigen Signalverlauf bestimmt wird.

2. Verfahren nach Anspruch 1, **dadurch gekennzeichnet, dass** aus der $B_0$-Karte (61) eine Suszeptibilitätskarte (62) abgeleitet wird.

3. Verfahren nach Anspruch 1 oder 2, **dadurch gekennzeichnet, dass** aus der $B_0$-Karte (61) die niederfrequenten Feldänderungen beseitigt werden, um eine Suszeptibilitätskarte (62) zu erhalten.

4. Verfahren nach einem der vorangehenden Ansprüche, **dadurch gekennzeichnet, dass** aus den Signalverläufen $B_1$-Werte ermittelt werden.

5. Verfahren nach einem der vorangehenden Ansprüche, **dadurch gekennzeichnet, dass** bei der Aufnahme des Magnetresonanzdatensatzes (54) wenigstens zwei Abschnitte (29, 30, 31, 32, 33, 34, 35, 36, 37, 38, 39) vorhanden sind und in wenigstens einem Abschnitt (30, 33, 36) Bilddatensätze (55, 56, 57) mit einer TrueFISP-Messsequenz (22) und in wenigstens einem Abschnitt (31, 34, 37) Bilddatensätze (55, 56, 57) mit einer FLASH-Messsequenz (11) aufgenommen werden.

6. Verfahren nach Anspruch 5, **dadurch gekennzeichnet, dass** in wenigstens einem Abschnitt (29, 32, 35, 38) Bilddatensätze (55, 56, 57) mit einer FISP-Messsequenz (18) aufgenommen werden.

7. Verfahren nach einem der vorangehenden Ansprüche, **dadurch gekennzeichnet, dass** die gemessenen Signalverläufe jeweils zur Bestimmung des $B_0$-Wertes oder des $B_0$-Wertes und wenigstens eines weiteren Parameterwertes mit simulierten Signalverläufen verglichen werden.

8. Verfahren nach Anspruch 7, **dadurch gekennzeichnet, dass** bei der Simulation der Signalverläufe nur ein reduzierter $B_0$-Wertebereich abgedeckt wird.

9. Verfahren nach Anspruch 8, **dadurch gekennzeichnet, dass** der $B_0$-Wertebereich auf Werte von $-(1/T_R)/2$ bis $+(1/T_R)/2$ eingeschränkt ist.

10. Verfahren nach einem der Ansprüche 8 oder 9, **dadurch gekennzeichnet, dass** die durch den Vergleich ermittelten Referenz-$B_0$-Werte nach Abschluss des Vergleichs mit den simulierten Signalverläufen auf $B_0$-Werte außerhalb des reduzierten Bereichs erweitert werden.

11. Verfahren nach Anspruch 10, **dadurch gekennzeichnet, dass** eine Erweiterungs-$B_0$-Karte berechnet wird, und durch Vergleich der Referenz-$B_0$-Karte mit der Erweiterungs-$B_0$-Karte bildelementweise Phaseninformationen ermittelt werden, um die $B_0$-Werte auf einen $B_0$-Wertebereich zu transformieren, der Werte außerhalb des reduzierten $B_0$-Wertebereichs enthält.

12. Verfahren nach einem der vorangehenden Ansprüche, **dadurch gekennzeichnet, dass** aus den Signalverläufen jeweils ein $T_1$-Wert und ein $T_2$-Wert ermittelt wird.

13. Computerprogrammprodukt (10) für eine Steuerungseinrichtung (8) zur Steuerung einer Datenerzeugungseinheit, insbesondere einer Bilderzeugungseinheit, einer Magnetresonanzanlage (1) zum Durchführen eines Verfahrens gemäß einem der vorhergehenden Ansprüche.

14. Datenträger (9) für eine Steuerungseinrichtung (8) zur Steuerung einer Datenerzeugungseinheit, insbesondere Bilderzeugungseinheit, einer Magnetresonanzanlage (1) mit Daten zum Durchführen eines Verfahrens gemäß einem der Ansprüche 1 bis 12.

15. Magnetresonanzanlage (1) mit einer Steuerungseinrichtung (8), **dadurch gekennzeichnet, dass** die Steuerungseinrichtung (8) zur Durchführung eines Verfahrens nach einem der Ansprüche 1 bis 12 ausgebildet ist.

**Geänderte Patentansprüche gemäss Regel 137(2) EPÜ.**

1. Verfahren zur Erzeugung einer $B_0$-Karte (61) eines Untersuchungsbereiches (58) mit den Schritten:

   - Bereitstellen eines Magnetresonanzdatensatzes (54) mit mehreren Bilddatensätzen (55, 56, 57), wobei die Bilddatensätze (55, 56, 57) mit wenigstens zwei Messsequenzen (11, 18, 22) aufgenommen wurden und die einander entsprechenden Bildelemente (59) der Bilddatensätze (55, 56, 57) jeweils einen zeitabhängigen Signalverlauf darstellen, wobei
   - mehr als die Hälfte der Bilddatensätze ohne steady state aufgenommen wurde,
   - Erzeugen einer $B_0$-Karte (61) des Untersuchungsbereiches (59) aus den Bilddatensätzen (55, 56, 57), wobei der $B_0$-Wert eines Bildelementes (59) der $B_0$-Karte (61) aus dem zugehörigen Signalverlauf bestimmt wird, wobei
   - die gemessenen Signalverläufe jeweils zur Bestimmung des $B_0$-Wertes oder des $B_0$-Wertes und wenigstens eines weiteren Parameterwertes mit simulierten Signalverläufen verglichen werden, und wobei
   - bei der Simulation der Signalverläufe nur ein beschränkter $B_0$-Wertebereich abgedeckt wird,

   **dadurch gekennzeichnet, dass**

   - durch den Vergleich ermittelte Referenz-$B_0$-Werte nach Abschluss des Vergleichs mit den simulierten Signal-

verläufen auf $B_0$-Werte außerhalb des beschränkten Bereichs erweitert werden.

2. Verfahren nach Anspruch 1, **dadurch gekennzeichnet, dass** aus der $B_0$-Karte (61) eine Suszeptibilitätskarte (62) abgeleitet wird.

3. Verfahren nach Anspruch 1 oder 2, **dadurch gekennzeichnet, dass** aus der $B_0$-Karte (61) die niederfrequenten Feldänderungen beseitigt werden, um eine Suszeptibilitätskarte (62) zu erhalten.

4. Verfahren nach einem der vorangehenden Ansprüche, **dadurch gekennzeichnet, dass** aus den Signalverläufen $B_1$-Werte ermittelt werden.

5. Verfahren nach einem der vorangehenden Ansprüche, **dadurch gekennzeichnet, dass** bei der Aufnahme des Magnetresonanzdatensatzes (54) wenigstens zwei Abschnitte (29, 30, 31, 32, 33, 34, 35, 36, 37, 38, 39) vorhanden sind und in wenigstens einem Abschnitt (30, 33, 36) Bilddatensätze (55, 56, 57) mit einer TrueFISP-Messsequenz (22) und in wenigstens einem Abschnitt (31, 34, 37) Bilddatensätze (55, 56, 57) mit einer FLASH-Messsequenz (11) aufgenommen werden.

6. Verfahren nach Anspruch 5, **dadurch gekennzeichnet, dass** in wenigstens einem Abschnitt (29, 32, 35, 38) Bilddatensätze (55, 56, 57) mit einer FISP-Messsequenz (18) aufgenommen werden.

7. Verfahren nach einem der vorangehenden Ansprüche, **dadurch gekennzeichnet, dass** der $B_0$-Wertebereich auf Werte von $- (1/T_R)/2$ bis $+(1/T_R)/2$ eingeschränkt ist.

8. Verfahren nach einem der vorangehenden Ansprüche, **dadurch gekennzeichnet, dass** eine Erweiterungs-$B_0$-Karte berechnet wird, und durch Vergleich der Referenz-$B_0$-Karte mit der Erweiterungs-$B_0$-Karte bildelementweise Phaseninformationen ermittelt werden, um die Referenz-$B_0$-Werte auf einen $B_0$-Wertebereich zu transformieren, der Werte außerhalb des beschränkten $B_0$-Wertebereichs enthält.

9. Verfahren nach einem der vorangehenden Ansprüche, **dadurch gekennzeichnet, dass** aus den Signalverläufen jeweils ein $T_1$-Wert und ein $T_2$-Wert ermittelt wird.

10. Computerprogrammprodukt (10) für eine Steuerungseinrichtung (8) zur Steuerung einer Datenerzeugungseinheit, insbesondere einer Bilderzeugungseinheit, einer Magnetresonanzanlage (1) zum Durchführen eines Verfahrens gemäß einem der vorhergehenden Ansprüche.

11. Datenträger (9) für eine Steuerungseinrichtung (8) zur Steuerung einer Datenerzeugungseinheit, insbesondere Bilderzeugungseinheit, einer Magnetresonanzanlage (1) mit Daten zum Durchführen eines Verfahrens gemäß einem der Ansprüche 1 bis 9.

12. Magnetresonanzanlage (1) mit einer Steuerungseinrichtung (8), **dadurch gekennzeichnet, dass** die Steuerungseinrichtung (8) zur Durchführung eines Verfahrens nach einem der Ansprüche 1 bis 9 ausgebildet ist.

FIG 1

EP 3 550 318 A1

FIG 2

FIG 3

ACQ

$G_R$

$G_P$

$G_S$

t

EP 3 550 318 A1

FIG 4

22

ACQ

$G_R$

$G_P$

$G_S$

EP 3 550 318 A1

FIG 5

## FIG 6

## FIG 7

FIG 8

FIG 9

61

$B_0$max

0

59

60

58

62

Smax

0

59

60

58

Europäisches
Patentamt

European
Patent Office

Office européen
des brevets

# EUROPÄISCHER RECHERCHENBERICHT

**Nummer der Anmeldung**

EP 18 16 6126

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DONGYEOB HAN ET AL.: "Development of magnetic resonance fingerprinting (MRF) combined with FISP and multi-echo SPGR acquisition for proton density, T1, T2, T2* and field mapping", PROCEEDINGS OF THE INTERNATIONAL SOCIETY FOR MAGNETIC RESONANCE IN MEDICINE, ISMRM, 25TH ANNUAL MEETING AND EXHIBITION, HONOLULU, HI, USA, 22 APRIL - 27 APRIL 2017, Nr. 3711, 7. April 2017 (2017-04-07), XP040691279, | 1-3,5-15 | INV. G01R33/24 G01R33/561 G01R33/56 |
| Y | * das ganze Dokument * | 2-4,9,10 | |
| Y | GREGOR KÖRZDÖRFER ET AL.: "Contrast and Resolution Mixing for Magnetic Resonance Fingerprinting", PROCEEDINGS OF THE INTERNATIONAL SOCIETY FOR MAGNETIC RESONANCE IN MEDICINE, ISMRM, 25TH ANNUAL MEETING AND EXHIBITION, HONOLULU, HI, USA, 22 APRIL - 27 APRIL 2017, Nr. 1404, 7. April 2017 (2017-04-07), XP040688972, * das ganze Dokument * | 4 | |
| Y | KARSTEN SOMMER ET AL.: "Towards Judging the Encoding Capability of Magnetic Resonance Fingerprinting Sequences", PROCEEDINGS OF THE INTERNATIONAL SOCIETY FOR MAGNETIC RESONANCE IN MEDICINE, ISMRM, 24TH ANNUAL MEETING AND EXHIBITION, SINGAPORE, 07 MAY - 13 MAY 2016, Nr. 429, 22. April 2016 (2016-04-22), XP040681472, * das ganze Dokument * | 9,10 | |

**RECHERCHIERTE SACHGEBIETE (IPC)**

G01R

-/--

Der vorliegende Recherchenbericht wurde für alle Patentansprüche erstellt

| Recherchenort | Abschlußdatum der Recherche | Prüfer |
|---|---|---|
| München | 24. Oktober 2018 | Durst, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 18 16 6126

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | E. MARK HAACKE ET AL: "Susceptibility weighted imaging (SWI)", MAGNETIC RESONANCE IN MEDICINE, Bd. 52, Nr. 3, 1. September 2004 (2004-09-01), Seiten 612-618, XP055096125, ISSN: 0740-3194, DOI: 10.1002/mrm.20198 * section "MATERIALS AND METHODS" * ----- | 2,3 | |
| A | Simone Coppo et al.: "Overview of Magnetic Resonance Fingerprinting", , 1. Februar 2016 (2016-02-01), XP055380755, Gefunden im Internet: URL:http://clinical-mri.com/wp-content/upl oads/2016/04/Gulani_MRF_MAGNETOM_Flash_ISM RM_2016.pdf [gefunden am 2017-06-12] * Abbildungen 2,3 * * Abschnitt "Acquisition sequence" * ----- | 1-15 | |
| T | GREGOR KÖRZDÖRFER ET AL: "Magnetic resonance field fingerprinting", MAGNETIC RESONANCE IN MEDICINE., 15. Oktober 2018 (2018-10-15), XP055516998, US ISSN: 0740-3194, DOI: 10.1002/mrm.27558 * das ganze Dokument * ----- -/-- | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 24. Oktober 2018 | Durst, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................
& : Mitglied der gleichen Patentfamilie, übereinstimmendes Dokument

EPO FORM 1503 03.82 (P04C03)

| Europäisches Patentamt / European Patent Office / Office européen des brevets | **EUROPÄISCHER RECHERCHENBERICHT** | **Nummer der Anmeldung**<br>EP 18 16 6126 |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | RICCARDO LATTANZI ET AL: "Phase unwinding for dictionary compression with multiple channel transmission in magnetic resonance fingerprinting",<br>MAGNETIC RESONANCE IMAGING,<br>Bd. 49, 24. Dezember 2017 (2017-12-24),<br>Seiten 32-38, XP055518025,<br>TARRYTOWN, NY, US<br>ISSN: 0730-725X, DOI:<br>10.1016/j.mri.2017.12.015<br>* Abbildung 1 *<br>* Abschnitt "2.1 Pulse sequence" *<br>----- | 1-15 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 24. Oktober 2018 | Durst, Markus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

    .................................................
& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **HAACKE et al.** Quantitative susceptibility mapping: current status and future directions. *Magnetic Resonance Imaging,* 2015, vol. 33, 1-33

- **MA D. et al.** Magnetic resonance fingerprinting. *Nature,* 2013, vol. 495, 187-193